(19) Europäisches Patentamt
European Patent Office
Office européen des brevets

(11) **EP 4 535 363 A1**

(12) **EUROPEAN PATENT APPLICATION**
published in accordance with Art. 153(4) EPC

(43) Date of publication:
**09.04.2025 Bulletin 2025/15**

(21) Application number: **23835612.5**

(22) Date of filing: **07.07.2023**

(51) International Patent Classification (IPC):
***G16B 30/10*** (2019.01)

(52) Cooperative Patent Classification (CPC):
**G16B 30/10**

(86) International application number:
**PCT/JP2023/025234**

(87) International publication number:
**WO 2024/010081 (11.01.2024 Gazette 2024/02)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **08.07.2022 JP 2022110810**

(71) Applicant: **Kumamoto University**
**Kumamoto-shi**
**Kumamoto 860-8555 (JP)**

(72) Inventors:
• **SATO Yorifumi**
  **Kumamoto-shi, Kumamoto 860-8555 (JP)**
• **UCHIYAMA Yoshikazu**
  **Kumamoto-shi, Kumamoto 860-8555 (JP)**

(74) Representative: **Isern Patentes y Marcas S.L.**
**Avda. Diagonal, 463 Bis, 2°**
**08036 Barcelona (ES)**

(54) **HIGH-PRECISION DIAGNOSTIC SYSTEM USING MULTI-ITEM SIMULTANEOUS MEASUREMENT DATA, HIGH-PRECISION DIAGNOSTIC METHOD, AND PROGRAM**

(57) [Problem] To provide a high-precision diagnostic system that uses multi-item simultaneous measurement data, classifies known mutations and unknown mutations with high precision, analyzes and visualizes classified results, and then visually displays a basis for a conclusion.

[Solution] A high-precision determination system 1 comprises: an acquisition unit that acquires at least multi-item simultaneous measurement data for an unknown sample and multi-item simultaneous measurement data for a known sample; a classifying unit that analyzes and quantifies similarity between the acquired multi-item simultaneous measurement data for the unknown sample and multi-item simultaneous measurement data for the known sample, classifies an unknown sample having a different trend than a known sample, and generates classification data; a determination unit that performs dimensional compression of the generated classification data to generate a two-dimensional scatter plot as determination data; a storage unit that stores at least the unknown multi-item simultaneous measurement data, the known multi-item simultaneous measurement data, the classification data, and the determination data; and a provision unit that provides at least the stored various types of data.

```
                    COMPUTER 2 START

         ACQUIRE MULTI-ITEM SIMULTANEOUS
         MEASUREMENT DATA AND BASE SEQUENCE DATA    S301

              GENERATE LEARNING MODEL              S302

                                                   S303
                 KNOWN MUTANT TYPE?

      S303a      YES              NO       S303b
      GENERATE MULTI-ITEM      GENERATE MULTI-ITEM
      MUTATION SITE            MUTATION SITE
      PATTERN DATA            PATTERN DATA

            GENERATE DIAGNOSTIC DATA               S304

                   STORE DATA                      S305

                  PROVIDE DATA                     S306

                  COMPUTER 2 END
```

**FIG. 3**

EP 4 535 363 A1

## Description

### Technical field

The present disclosure relates to a high-precision diagnostic method and program using multi-item simultaneous measurement data for detecting and visualizing known mutation and unknown mutation.

### Background

For example, the most comprehensive method for detecting base sequence mutation, i.e., genetic mutation, is to analyze a genome by using Whole Genome Sequencing (WGS). However, WGS requires a sophisticated analytical device, technology, and expensive reagent, and therefore there is a need for a technology to quantitatively detect and diagnose genetic mutation easily and quickly.

One of the technologies to quantitatively detect a mutation site easily and quickly is, for example, a multi-item simultaneous gene-related test using a DNA microarray. The presence or the absence of known genetic mutation can be detected as multi-item simultaneous measurement data for each mutation site through a multi-item simultaneous gene-related test.

Conventionally, the presence or the absence of a mutation site is detected from the above-mentioned multi-item simultaneous measurement data, and whether or not there is a matched known typical mutation pattern is judged to diagnose whether the mutation is known or unknown.

For example, Patent Document 1 discloses an analysis method for calculating the pattern and the frequency of a mutation by comparing each read sequence (sequence information of a base sequence to be sequenced) with a reference sequence to detect a base mutation from each read sequence and analyzing the result.

In addition, Patent Document 2 discloses an idea of giving priority to detecting a mutation in the broader region of a sequence and analyzing the mutation pattern to analyze a tendency in genetic mutation within the broader region of the sequence.

In addition, Non-patent Document 1 discloses a method in which the evolutionary distance from the gene of a virus is determined as a p-distance, and the p-distance is distributed in a Euclidean space by using a Multi-Dimensional Scaling method.

### Document in the existing art

Patent Literature

Patent Document 1: JP 2018-130114 A
Patent Document 1: JP 2021-126125 A

Non-patent Literature

Non-Patent Document 1: Special Feature: Report on Research Activities of Students - Participation in Domestic and International Conferences 22, online, retrieved on May 20, 2022, Internet, https://www.rikou.ryukoku.ac.jp/ journal/-journal69/RJ69S-19.pdf

### Summary

The technical problem solved by the disclosure

The technologies described in Patent Documents 1 and 2, and Non-Patent Document 1 can discriminate known genetic mutation, detect unknown genetic mutation, and visualize the evolutionary distance of differences in genetic mutation.

However, those technologies cannot highly accurately show which known mutation unknown mutation is close to or visually demonstrate the basis for determination of the analysis result that the mutation is unknown mutation, after categorizing and visualizing known mutation and unknown mutation.

The present disclosure provides a high-precision diagnostic system using multi-item simultaneous measurement data for highly accurately categorizing known mutation and unknown mutation, analyzing and visualizing the categorized result, and visually demonstrating the base for determination.

Solution for solving the technical problem

According to a first aspect of the present disclosure, the high-precision determination system using multi-item simultaneous measurement data for detecting and visualizing a known sample and an unknown sample, includes:

an acquisition unit that acquires at least multi-item simultaneous measurement data on an unknown sample and multi-item simultaneous measurement data on a known sample;

a categorizing unit that analyzes and quantifies similarity between the acquired multi-item simultaneous measurement data on the unknown sample and the acquired multi-item simultaneous measurement data on the known sample, categorizes an unknown sample that differs from a known sample in tendency, and generates categorization data;

a determination unit that dimensionally compresses the generated categorization data and generates a two-dimensional scatter diagram as determination data;

a storage unit that stores at least the multi-item simultaneous measurement data on the unknown sample, the multi-item simultaneous measurement data on the known sample, the categorization data, and the determination data; and

a provisioning unit that provides at least the stored data.

According to the first aspect of the present disclosure, the high-precision determination system using multi-item simultaneous measurement data for detecting and visualizing a known sample and an unknown sample acquires at least multi-item simultaneous measurement data on an unknown sample and multi-item simultaneous measurement data on a known sample; analyzes and quantifies similarity between the acquired multi-item simultaneous measurement data on the unknown sample and the acquired multi-item simultaneous measurement data on the known sample, categorizes an unknown sample that differs from a known sample in tendency, and generates categorization data; dimensionally compresses the generated categorization data and generates a two-dimensional scatter diagram as determination data; stores at least the multi-item simultaneous measurement data on the unknown sample, the multi-item simultaneous measurement data on the known sample, the categorization data, and the determination data; and provides at least the stored data.

The first aspect of the present disclosure is categorized into a high-precision diagnostic system, but a high-precision diagnostic method and a program also have the same actions and effects.

According to a second aspect of the present disclosure, the high-precision diagnostic system according to the first aspect of the present disclosure, which is:

a high-precision diagnostic system using multi-item simultaneous measurement data for detecting and visualizing known mutation and unknown genetic mutation, the multi-item simultaneous measurement data and the known data being related to a basic sequence, includes:

an acquisition unit that acquires at least the multi-item simultaneous measurement data and a known basic sequence data;

a learning model generation unit that categorizes the acquired multi-item simultaneous measurement data and basic sequence data and detects unknown genetic mutation that differ from known genetic mutation in tendency by using the local outlier factor method, and generates a learning model that generates multi-item mutation site pattern data;

a diagnostic unit that generates a two-dimensional scatter diagram as diagnostic data from the generated multi-item mutation site pattern data and from learning data for the learning model based on the learning model by using the Multi-Dimensional Scaling method;

a storage unit that stores at least the multi-item simultaneous measurement data, the known data, the multi-item mutation site pattern data, and the diagnostic data; and

a provisioning unit that provides at least the stored data.

According to the second aspect of the present disclosure, in the high-precision diagnostic system according to the first aspect of the present disclosure, the categorizing unit analyzes and quantifies similarity between the acquired multi-item simultaneous measurement data of the unknown sample and the acquired multi-item simultaneous measurement data of the known sample by using the k-nearest neighbor method (k-Nearest Neighbor, kNN), categorizes an unknown sample that differs from the known sample in tendency by using the local outlier factor (LOF) method, and the determination unit generates a two-dimensional scatter diagram as determination data from the categorization data by using the Multi-Dimensional Scaling (MDS) method.

According to a third aspect of the present disclosure, in the high-precision diagnostic system according to the first or the second aspect of the present disclosure, the multi-item simultaneous measurement data is data related to a nucleic acid sequence obtained from the unknown sample and the known sample.

According to a fourth aspect of the present disclosure, in the high-precision diagnostic system according to the first or the second aspect of the present disclosure, a known new coronavirus variant and an unknown new coronavirus variant are determined.

According to the fourth aspect of the present disclosure, in the high-precision diagnostic system of the first or the second aspect of the present disclosure, a known new coronavirus variant and a new coronavirus variant are diagnosed.

According to a fifth aspect of the present disclosure, in the high-precision diagnostic system according to the third aspect of the present disclosure, the unknown sample and the known sample are cancer cells.

According to a sixth aspect of the present disclosure, in the high-precision diagnostic system according to the third aspect of the present disclosure, the multi-item simultaneous measurement data is data indicating a measured abundance of a specific protein for determining clinical condition.

According to a seventh aspect of the present disclosure, the high-precision diagnostic system according to the first aspect of the present disclosure includes a learning model generation unit that generates a learning model that improves categorization accuracy by recognizing one or more quantified items as one or more patterns; and

a pattern data generation unit that generates pattern data from the categorization data based on the learning model, in which
the storage unit stores at least the pattern data and the learning model, and the providing unit provides at least the pattern data.

Technical effect

The present disclosure can provide a high-precision diagnostic system that has capability of highly accurately detecting known mutation and unknown mutation from multi-item simultaneous measurement data, analyzing and visualizing the detection result, and visually demonstrating the base for determination.

**Brief description of the drawings**

FIG. 1 is a schematic diagram of the high-precision diagnostic system 1.
FIG. 2 is a configuration diagram of the high-precision diagnostic system 1.
FIG. 3 is a flowchart showing the procedure of the high-precision diagnosis process executed by the computer 2 of the high-precision diagnostic system 1.
FIG. 4 is an example display screen of multi-item mutation site pattern data generated by the computer 2, which is displayed by the user terminal 3.
FIG. 5 is an example display screen of diagnostic data generated by the computer 2, which is displayed by the user terminal 3.

**Detailed description of the embodiments**

The best mode for embodying the present disclosure is described below with reference to the attached drawings. However, this is merely an example, and the technical scope of the present disclosure is not limited thereto.

Overview of high-precision diagnostic system1

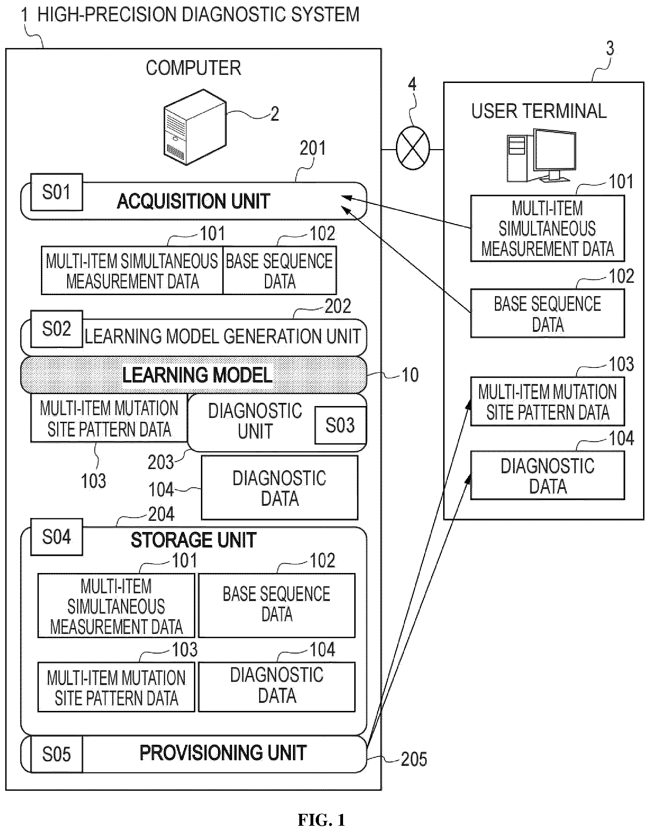

FIG. 1 is a diagram to explain an overview of the high-precision diagnostic system 1.

As shown in FIG. 1, the high-precision diagnostic system 1 is, for example, a computer system for use in the diagnosis of genetic mutation, which is a system including at least a computer 2 and a user terminal 3. The computer 2 and the user terminal 3 can communicate with each other through a network 4.

The computer 2 of the high-precision diagnostic system 1 is a computer for controlling the high-precision diagnostic system 1, which may be realized in, for example, one or more physical computers or a virtual device such as a cloud computer.

The user terminal 3 of the high-precision diagnostic system 1 is a terminal for transmitting and receiving multi-item simultaneous measurement data 101, base sequence data 102, multi-item mutation site pattern data 103, and diagnostic data 104 to and from the computer 2, which may be a personal computer, a laptop computer, a mobile terminal such as a smart phone or a tablet terminal, or a wearable terminal such as a head-mounted display such as smart glasses or a smart watch.

First, the acquisition unit 201 of the computer 2 acquires at least multi-item simultaneous measurement data 101 and base sequence data 102 from the user terminal 3 (step S01). In the present disclosure, the multi-item simultaneous measurement data 101 shall be data acquired by a multi-item simultaneous gene-related test, for example, a microarray. The multi-item simultaneous measurement data 101 may be substituted with data such as 0 or 1 representing the absence or the presence, respectively, of mutation, in which a threshold may be set for each genetic mutation site. Alternatively, the multi-item simultaneous measurement data 101 may be quantitatively substituted with the mutant type of a mutation site. The base sequence data 102 shall include, for example, at least the base sequence data of a wild type (WT) strain with a genetic type which an organism or a microorganism inherently has for a mutant with a mutated gene, and the base sequence data of a mutant (MUT) strain with a known mutated gene. These base sequence data are genomically analyzed by using WGS. The base sequence data 102 may be substituted with quantifiable data for each genetic mutation site according to the multi-item simultaneous measurement data 101. The timing of acquisition of such data is not limited, nor is the data format.

Next, the learning model generation unit 202 of the computer 2 categorizes known genetic mutation from the acquired at least multi-item simultaneous measurement data 101 and base sequence data 102 by using kNN, detects unknown genetic mutation that differs from known genetic mutation in tendency by using LOF, and generates a learning model 10 that generates multi-item mutation site pattern data 103 (step S02). The multi-item mutation site pattern data 103 may be, for example, a barcode pattern in which a mutation site is quantitatively substituted with the mutant type of a mutation site as a panel. If the data indicates known genetic mutation, the multi-item mutation site pattern data 103 may be generated by linking the name of a known mutant type with reference to the base sequence data 102. If the data indicates unknown genetic mutation, the multi-item mutation site pattern data 103 may be generated by linking a predefined name such as Atypical.

Next, the diagnostic unit 203 of the computer 2 generates a two-dimensional scatter diagram generated by using MDS from the multi-item mutation site pattern data 103 generated based on the learning model 10 and the learning data for the learning model 10, as diagnostic data 104 (step S03). The diagnostic data 104 is data visualizing the basis for determination of the learning model 10 in a two-dimensional scatter diagram with the difference from known mutation as distance, for example, if the multi-item mutation site pattern data 103 generated by the learning model 10 indicates unknown mutation.

Next, the storage unit 204 of the computer 2 stores at least the acquired multi-item simultaneous measurement data 101, the acquired base sequence data 102, the generated multi-item mutation site pattern data 103, and the diagnostic data 104 in the computer 2 (step 04).

Finally, the provisioning unit 205 of the computer 2 provides the data stored by the storage unit 204 through at least the user terminal 3 (step 05).

The above is an overview of the high-precision diagnostic system 1.

Configuration of high-precision diagnostic system 1

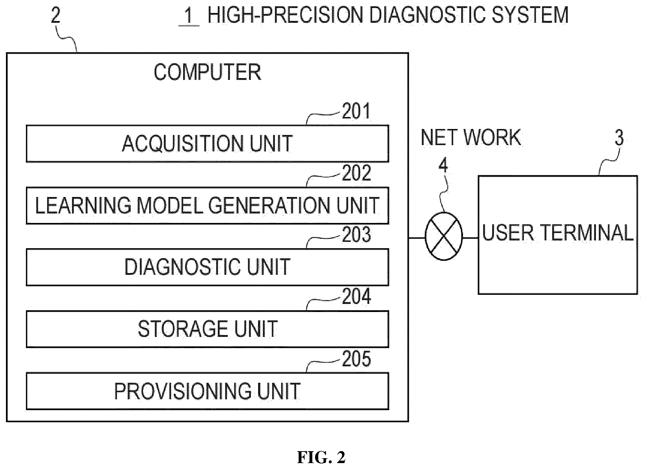

FIG. 2 is a diagram to explain the configuration of the high-precision diagnostic system 1.

As shown in FIG. 2, the computer 2 of the high-precision diagnostic system 1 includes a control unit (not shown) equipped with a CPU (Central Processing Unit), a GPU (Graphics Processing Unit), a RAM (Random Access Memory), and a ROM (Read Only Memory) and a communication unit (not shown) equipped with a device for enabling communication with other terminals, devices, etc., such as a Wi-Fi® (Wireless-Fidelity)-compliant device in accordance with IEEE802.11.

The computer 2 also includes a memory unit (not shown) as a data storage unit by means of a hard disk, a semiconductor memory, a recording medium, a memory card, etc. The data storage unit may exist outside where network communication is enabled.

In the computer 2, the control unit realizes an acquisition unit 201, a learning model generation unit 202, a diagnosis unit 203, a storage unit 204, and a provisioning unit 205 in cooperation with the memory unit and the communication unit and the memory unit by a predetermined program.

The user terminal 3 is an electronic device such as a computer, a smartphone, or a tablet terminal, which is data-communicatively connected with the above-mentioned computer 2 through a network 4. The number of user terminals 3 is not necessarily one and may be more than one.

The user terminal 3 includes a terminal control unit (not shown) equipped with a CPU, GPU, RAM, ROM, etc., in the same way as the control unit in the above-mentioned computer 2. The user terminal 3 also includes a communication unit (not shown) equipped with a device for enabling communication with other terminals, devices, etc. The user terminal 3 also includes an input/output unit (not shown) equipped with an input/output device, etc., for inputting and outputting data. The user terminal 3 also includes a memory unit (not shown) as a data storage unit (not shown) by means of a hard disk, a semiconductor memory, a recording media, a memory card, etc.

The above is the configuration of the high-precision diagnostic system 1.

Genetic mutation high-precision diagnostic process

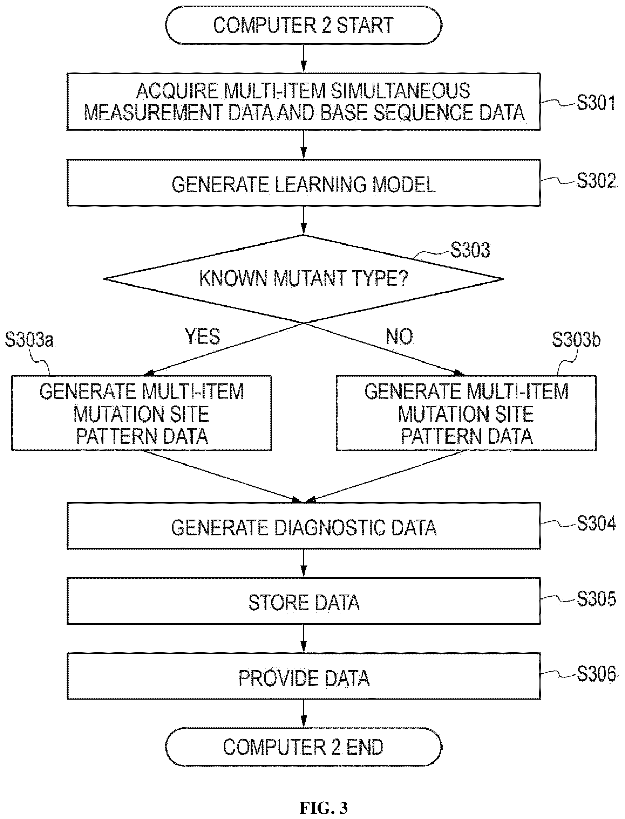

FIG. 3 is a flowchart diagram showing the procedure of the genetic mutation high-precision diagnosis process executed by the computer 2 of the high-precision diagnostic system 1. The genetic mutation high-precision diagnostic process executed by the computer 2 is explained below with reference to Fig. 3.

First, the acquisition unit 201 of the computer 2 acquires at least multi-item simultaneous measurement data 101 and base sequence data 102 (step S301). The data may be already stored in the computer 2 or may be acquired by data-communicatively connecting with the user terminal 3 through a network 4 such as a public line network.

Next, the learning model generation unit 202 of the computer 2 categorizes known genetic mutation from the acquired at least multi-item simultaneous measurement data 101 and base sequence data 102 by using kNN, detects unknown genetic mutation that differs from known genetic mutation in tendency by using LOF, and generates a learning model 10 that generates multi-item mutation site pattern data 103 (step S302).

Next, the learning model 10 of the computer 2 categorizes known genetic mutation from the multi-item simultaneous measurement data 101, detects unknown genetic mutation, and generates the multi-item mutation site pattern data 103 (step S303).

The method of categorizing known genetic mutation by using kNN is explained in detail below, using a new coronavirus variant as an example.

The multi-item simultaneous measurement data 101 is data acquired from a multi-item simultaneous gene-related test for a new coronavirus variant, in which a threshold is set for each genetic mutation site. The multi-item simultaneous measurement data 101 is quantifiable data according to the mutant type of the mutation site. For example, the mutation site is shown as a panel, and the output value varies depending on the shade of the color of the panel. The panel detected in multiple items is substituted with a barcode pattern.

The base sequence data 102 is base sequence data at least including base sequence data acquired from the WT of a new coronavirus variant or base sequence data of a mutant type with a known mutated gene, which is substituted with quantifiable data for each genetic mutation site according to the multi-item simultaneous measurement data 101.

The categorization of known genetic mutation first represents $\alpha_n$ expressed by the output value (continuous value) of the nth mutation site. In this case, the output of a certain mutant type can represent the vector $\alpha = (\alpha_1, \alpha_2, ..., \alpha_n)$ expressed by a vector in a n-dimensional space. Since the magnitude of the components of the vector varies with the amount of virus, normalization is performed by dividing the vector $\alpha$ by the magnitude of the vector. Accordingly, each case is placed on a unit circumference of n dimensions, and each case is categorized depending on which direction the case faces from the origin. If the barcode patterns on the same panel have different color intensities (different magnitudes of the output values), but the barcode patterns on the panel are the same, the genetic mutation is categorized as a same mutant type because the cases face the same direction.

In the learning performed by the learning model generation unit 202, the above-mentioned base sequence data 102 is used as teacher data for learning, the learning data is placed on an n-dimensional space and linked to the name of a known mutant type, and a learning model 10 is generated.

When newly acquired multi-item simultaneous measurement data 101 is categorized as test data (vector $\beta = (\beta_1, \beta_2, ..., \beta_n)$), the Euclidean distance between the ith learning data (vector $\alpha_i$) and the test data (vector $\beta$) is calculated, the learning data with the closest distance is determined and linked to the name of a mutant type in the learning data, and multi-item mutation site pattern data 103 is generated (Step S303a).

The method of detecting unknown genetic mutation by using LOF is explained in detail below, using a new coronavirus variant as an example.

First, the reachable distance from x to x' is defined by Formula 1 below. In the formula, $x^{(k)}$ represents the kth closest case to x in the learning data $\{x_i\}^n{}_i = 1$. The local reachable density of x is defined as Formula 2 below. Using this local reachable density, the LOF of x is defined by Formula 3 below.

LOFk(x) is the ratio of the average local reachable density of $x^{(i)}$ to the local reachable density of x. If the density around the learning data $x^{(i)}$ is high, but the density around the test data x is low, the LOF value increases. This case is determined as an unknown pattern not existing in the learning data is detected as unknown genetic mutation, and multi-item mutation site pattern data 103 is generated (step S303b).

$$\text{Formula 1}$$

$$\mathrm{RD}_k(x, x^{'}) = \max\{\|x - x^{(k)}\|, \|x - x'\|\}$$

Formula 2

$$\mathrm{LRD}_k(x) = \left( \frac{1}{k} \sum_{i=1}^{k} \mathrm{RD}_k(x^{(k)}, x) \right)^{-1}$$

Formula 3

$$\mathrm{LOF}_k(x) = \frac{\frac{1}{k} \sum_{i=1}^{k} \mathrm{LRD}_k(x^{(i)})}{\mathrm{LRD}_k(x)}$$

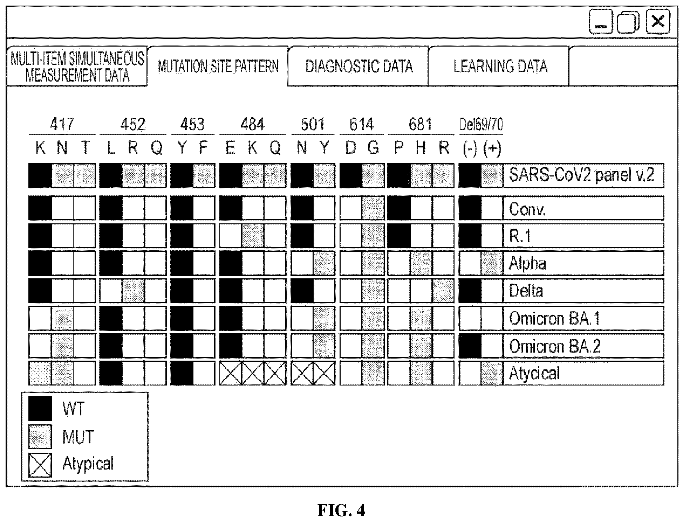

FIG. 4 is an example display screen of the multi-item mutation site pattern data 103 generated by the computer 2, which is displayed by the user terminal 3 for a new coronavirus variant. As shown in FIG. 4, the multi-item mutation site pattern data 103 generated by the computer 2 is displayed as the mutation site pattern, for example, a heatmap which is a type of visualized graph expressing the individual values of two-dimensional data by using colors and shades after a known new coronavirus variant such as a Delta variant is categorized and an unknown new coronavirus variant is detected.

Thus, it is possible to categorize known genetic mutation and detect unknown genetic mutation, but it is difficult to demonstrate the basis for the reason why the learning model 10 is so determined. As the result, the result presented by the learning model 10 is hardly trusted and utilized.

Then, the diagnostic unit 203 of the computer 2 generates a two-dimensional scatter diagram visualized from data distribution by using MDS from the multi-item mutation site pattern data 103 and from the learning data for the learning model 10, as diagnostic data 104 (step S304).

The method of visualizing data distribution by using MDS is explained in detail below, using a new coronavirus variant as an example.

Since the output value of the multi-item simultaneous measurement data 101 is n-dimensional data, each case is distributed as a single point in an n-dimensional space. If the n-dimensional data can be dimensionally reduced to two-dimensional data (or three-dimensional data), the dimensional data can be displayed in a scatter diagram, allowing the user to intuitively determine which known mutation the test data is close to or whether the test data is unknown mutation. Thus, the basis for determination of the result presented by the learning model 10 can be used. Then, dimensional aggregation is performed by using MDS. MDS configures a new axis in the following procedure.

First, the distance matrix $d_{ij}$, which is made up of the Euclidean distances between the input i and the input j, is determined, and then the transformation matrix $Z_{ij}$, by which the origin moves to become the center of gravity of n input data, is determined (Formula 4).

Formula 4

$$z_{ij} = -\frac{1}{2} \left( d_{ij}^2 - \sum_{i=1}^{n} \frac{d_{ij}^2}{n} - \sum_{j=1}^{n} \frac{d_{ij}^2}{n} + \sum_{i=1}^{n} \sum_{j=1}^{n} \frac{d_{ij}^2}{n^2} \right)$$

Next, a new coordinate point is determined as coordinate values on the axes given by the eigenvector of the matrix $Z_{ij}$. MDS is a linear transformation that maintains the Euclidean distance between data so that the relative positional relationship of each case in an n-dimensional space can be interpreted to be reproduced in a two-dimensional space.

The learning data is used to determine a conversion equation for dimensional reduction of n-dimensional data to two-dimensional data, and the conversion equation is then applied to the test data to determine the position of the test data in a two-dimensional space. Since the output value of the multi-item simultaneous measurement data 101 is represented as a barcode pattern, for example, as (1,0) or (0,1), each case is distributed along an axis in a linear independent relationship in an n-dimensional space.

Since MDS is a method of determining two new axes by means of eigenvectors in an orthonormal relationship in a way that maintains the Euclidean distance for each case, the key to the present disclosure is that MDS is a dimensional reduction method that takes into account the mathematical properties of the barcode pattern of the multi-item simultaneous measurement data 101.

In addition, in t-distributed Stochastic Neighbor Embedding, (t-SNE), which is frequently used as a dimensional reduction method, a new axis is formed each time when new test data is entered, so that learning and testing cannot be performed separately. Thus, MDS, which can add the distribution of test data over two-dimensional distribution of fixed known mutation, is considered to be superior to t-SNE.

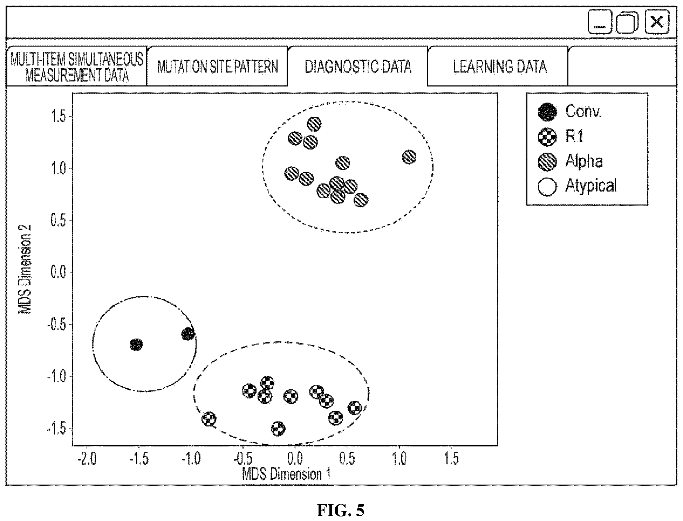

FIG. 5 is an example display screen of the diagnostic data 104 generated by the computer 2, which is displayed by the user terminal 3 for a new coronavirus variant. As shown in FIG. 5, the diagnostic data 104 generated by the computer 2, which is generated in a two-dimensional scatter diagram, as diagnostic data from the multi-item mutation site pattern data 103 after a known new coronavirus variant such as a Delta valiant, etc., is categorized and after an unknown new coronavirus variant is detected, can visually demonstrate the validity of the multi-item mutation site pattern data 103 generated based on the learning model.

The above is the high-precision diagnostic process.

Such a high-precision diagnostic process can provide a high-precision diagnostic system using multi-item simultaneous measurement data from multi-item simultaneous gene-related test for highly accurately categorizing known genetic mutation and unknown genetic mutation, analyzing and visualizing the categorized result, and visually demonstrating the base for determination.

The high-precision diagnostic system 1 is useful as a diagnostic test not only for the above-mentioned new coronavirus variant but also for other diseases, for example, by detecting genetic mutation in a cancer cell or by detecting a specific protein.

Diagnosis by detection of genetic mutation in cancer cell

The system can be used as an AI system to suggest optimal treatment for cancer. In recent years, genetic abnormalities related to cancer growth have been revealed one after another. Currently, after testing for genetic mutation in a cancer cell, treatment with a molecularly targeted drug which can be expected to have a significant effect is administered if the conditions are right. For example, EGFR genetic mutation, ALK fusion genes, HER2 genetic mutation, MET genetic mutation, ROS1 fusion genes, BRAF genetic mutation, RET fusion genes, and RAS genetic mutation are known as genetic abnormalities related to lung adenocarcinoma. Genomic medicine, in which genetic mutation in a cancer cell is combined with a molecularly targeted drug, is used for diagnosis and treatment. For example, if there is only EGFR genetic mutation, the molecularly targeted drug gefitinib is likely to be dramatically effective.

The result of a test for the presence or the absence of genetic mutation in a cancer cell can be thought in the same way as the output of the panel pattern in the present disclosure. Therefore, by analyzing information on the presence or the absence of genetic mutation in a cancer cell by using the same algorithm as the present disclosure, it is possible to select an effective molecularly targeted drug and to construct and use an AI system to suggest optimal treatment. It is also possible to predict that the case will not respond to a molecularly targeted drug if the result of the test is different from a known pattern.

Detection of specific protein

Data such as the Bio-Plex™ Suspension Array System, which uses the bind of 100 different color-coded beads that specifically conjugate to different proteins, provides multi-item information of up to 100 different biological components (e.g., proteins, nucleic acids) in the same way as multi-item simultaneous measurement data of a new coronavirus variant. The method of the present disclosure is expected to be useful in the diagnosis of inflammatory condition by utilizing multi-item data on an inflammatory cytokine, which can be acquired by using a panel of the inflammatory cytokine.

The embodiments of the present disclosure are described above, but the present disclosure is not limited to these embodiments. The effects described in the embodiments of the present disclosure are only a list of the most suitable effects arising from the present disclosure, and the effects of the present disclosure are not limited to those described in the embodiments of the present disclosure.

Description of reference numerals

1: High-precision diagnostic system, 2: Computer, 3: User terminal, 4: Network

**Claims**

1. A high-precision determination system using multi-item simultaneous measurement data for detecting and visualizing a known sample and an unknown sample, comprising:

an acquisition unit that acquires at least multi-item simultaneous measurement data on an unknown sample and multi-item simultaneous measurement data on a known sample;

a categorizing unit that analyzes and quantifies similarity between the acquired multi-item simultaneous measurement data on the unknown sample and the acquired multi-item simultaneous measurement data on the known sample, categorizes an unknown sample that differs from a known sample in tendency, and generates categorization data;

a determination unit that dimensionally compresses the generated categorization data and generates a two-dimensional scatter diagram as determination data;

a storage unit that stores at least the multi-item simultaneous measurement data on the unknown sample, the multi-item simultaneous measurement data on the known sample, the categorization data, and the determination data; and

a provisioning unit that provides at least the stored data.

2. The high-precision determination system according to claim 1, wherein the categorizing unit analyzes and quantifies similarity between the acquired multi-item simultaneous measurement data of the unknown sample and the acquired multi-item simultaneous measurement data of the known sample by using the k-nearest neighbor method (k-Nearest Neighbor, kNN), categorizes an unknown sample that differs from the known sample in tendency by using the local outlier factor (LOF) method, and the determination unit generates a two-dimensional scatter diagram as determination data from the categorization data by using the Multi-Dimensional Scaling (MDS) method.

3. The high-precision determination system according to claim 1 or 2, wherein the multi-item simultaneous measurement data is data related to a nucleic acid sequence obtained from the unknown sample and the known sample.

4. The high-precision determination system according to claim 1 or 2, wherein a known new coronavirus variant and an unknown new coronavirus variant are determined.

5. The high-precision determination system according to claim 3, wherein the unknown sample and the known sample are cancer cells.

6. The high-precision determination system according to claim 1 or 2, wherein the multi-item simultaneous measurement data is data indicating a measured abundance of a specific protein for determining clinical condition.

7. The high-precision determination system according to claim 1, further comprising:

a learning model generation unit that generates a learning model that improves categorization accuracy by recognizing one or more quantified items as one or more patterns; and

a pattern data generation unit that generates pattern data from the categorization data based on the learning model, wherein

the storage unit stores at least the pattern data and the learning model, and the providing unit provides at least the pattern data.

8. The high-precision determination method executed by a computer that uses multi-item simultaneous measurement data for detecting and visualizing a known sample and an unknown sample, comprising the steps of:

acquiring at least multi-item simultaneous measurement data on an unknown sample and multi-item simultaneous measurement data on a known sample;

analyzing and quantifying similarity between the acquired multi-item simultaneous measurement data on the unknown sample and the acquired multi-item simultaneous measurement data on the known sample, categorizing an unknown sample that differs from a known sample in tendency, and generating categorization data;

dimensionally compressing the generated categorization data and generating a two-dimensional scatter diagram as determination data;

storing at least the multi-item simultaneous measurement data on the unknown sample, the multi-item simultaneous measurement data on the known sample, the categorization data, and the determination data; and

providing at least the stored data.

9. A program causing a computer that uses multi-item simultaneous measurement data for detecting and visualizing a known sample and an unknown sample to execute the steps of:

acquiring at least multi-item simultaneous measurement data on an unknown sample and multi-item simultaneous measurement data on a known sample;

analyzing and quantifying similarity between the acquired multi-item simultaneous measurement data on the unknown sample and the acquired multi-item simultaneous measurement data on the known sample, categorizing an unknown sample that differs from a known sample in tendency, and generating categorization data;

dimensionally compressing the generated categorization data and generating a two-dimensional scatter diagram as determination data;

storing at least the multi-item simultaneous measurement data on the unknown sample, the multi-item simultaneous measurement data on the known sample, the categorization data, and the determination data; and

providing at least the stored data.

1 HIGH-PRECISION DIAGNOSTIC SYSTEM

**FIG. 1**

1 HIGH-PRECISION DIAGNOSTIC SYSTEM

COMPUTER

ACQUISITION UNIT — 201

LEARNING MODEL GENERATION UNIT — 202

DIAGNOSTIC UNIT — 203

STORAGE UNIT — 204

PROVISIONING UNIT — 205

NET WORK

USER TERMINAL

**FIG. 2**

**FIG. 3**

FIG. 4

**FIG. 5**

## INTERNATIONAL SEARCH REPORT

| International application No. |
|---|
| **PCT/JP2023/025234** |

### A.  CLASSIFICATION OF SUBJECT MATTER

*G16B 30/10*(2019.01)i
FI:  G16B30/10

According to International Patent Classification (IPC) or to both national classification and IPC

### B.  FIELDS SEARCHED

Minimum documentation searched (classification system followed by classification symbols)

G16B30/10

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

### C.  DOCUMENTS CONSIDERED TO BE RELEVANT

| Category* | Citation of document, with indication, where appropriate, of the relevant passages | Relevant to claim No. |
|---|---|---|
| A | JP 2022-527316 A (GRAIL, INC) 01 June 2022 (2022-06-01)<br>claims 1, 2, 31-33, 35 | 1-9 |
| A | JP 2021-179867 A (DISUM CO LTD) 18 November 2021 (2021-11-18)<br>paragraphs [0016]-[0024], [0030], [0042]-[0044] | 1-9 |
| A | JP 2018-147202 A (UNIV AKITA PREFECTURAL) 20 September 2018 (2018-09-20)<br>paragraphs [0048]-[0049] | 1-9 |
| A | 松村 玲央, 多項空間スキャン統計量を用いた新型コロナウイルス型別構成の空間クラスター検出, 一般社団法人地理情報システム学会 講演論文集 [CD-ROM], 23 October 2020, vol. 29<br>pp. 1-6, (MATSUMURA, Reo. Detection of Spatial Clusters of Virus Clade Composition of the 2019 Novel Coronavirus Using Spatial Scan Statistics for Multinomial Data.), non-official translation (Lecture Proceedings of Geographic Information System Association (CD_ROM]) | 1-9 |
| A | US 2018/0336316 A1 (LIFE TECHNOLOGIES CORPORATION) 22 November 2018 (2018-11-22)<br>claims 1, 11-12 | 1-9 |

☐ Further documents are listed in the continuation of Box C.  ☑ See patent family annex.

| * Special categories of cited documents: | |
|---|---|
| "A" document defining the general state of the art which is not considered to be of particular relevance | "T" later document published after the international filing date or priority date and not in conflict with the application but cited to understand the principle or theory underlying the invention |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **07 September 2023** | **19 September 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

INTERNATIONAL SEARCH REPORT
Information on patent family members

International application No.

**PCT/JP2023/025234**

| Patent document cited in search report | | | Publication date (day/month/year) | Patent family member(s) | | | Publication date (day/month/year) |
|---|---|---|---|---|---|---|---|
| JP | 2022-527316 | A | 01 June 2022 | US | 2020/0318190 | A1 | |
| | | | | claims 1, 2, 31-33, 35 | | | |
| | | | | EP | 3947742 | A1 | |
| | | | | CN | 113710818 | A | |
| | | | | KR | 10-2021-0149052 | A | |
| JP | 2021-179867 | A | 18 November 2021 | (Family: none) | | | |
| JP | 2018-147202 | A | 20 September 2018 | (Family: none) | | | |
| US | 2018/0336316 | A1 | 22 November 2018 | EP | 3625714 | A1 | |
| | | | | CN | 110914911 | A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2018130114 A

- JP 2021126125 A

**Non-patent literature cited in the description**

- Special Feature: Report on Research Activities of Students - Participation. *Domestic and International Conferences*, 20 May 2022, vol. 22, https://www. rikou.ryukoku.ac.jp/ journal/journal69/RJ69S-19.pdf